# EUROPEAN PATENT SPECIFICATION

(11) **EP 2 878 302 B1**
(45) Date of publication and mention of the grant of the patent: **27.11.2019**
(21) Application number: 13823573.4
(22) Date of filing: 25.07.2013
(51) Int. Cl.: A61K 35/60, A61K 38/00, A61P 19/02

(54) **COMPOSITION FOR PREVENTING OR TREATING OSTEOARTHROSIS**
ZUSAMMENSETZUNG ZUR PRÄVENTION ODER BEHANDLUNG VON OSTEOARTHROSE
COMPOSITION DESTINÉE À LA PRÉVENTION OU AU TRAITEMENT DE L'OSTÉOARTHRITE

(30) Priority: 25.07.2012 JP 2012164723
(43) Date of publication of application: 03.06.2015
(73) Proprietor: Hirosaki University, Hirosaki-shi, Aomori 036-8560 (JP); Wakitani Medical Science Corporation, Osaka-shi, Osaka 540-0013 (JP); Sunstar Inc., Takatsuki-shi Osaka 569-1195 (JP)
(72) Inventor: KATO, Yoji, Hirosaki-shi Aomori 036-8560 (JP); WAKITANI, Shigeyuki, Osaka-shi Osaka 540-0013 (JP); HANADA, Yukako, Takatsuki-shi Osaka 569-1195 (JP); YAMAMOTO, Kazushi, Takatsuki-shi Osaka 569-1195 (JP)
(74) Representative: Müller-Boré & Partner Patentanwälte PartG mbB
(86) International application number: PCT/JP2013/070134
(87) International publication number: WO 2014/017570

(56) References cited:
- EP-A1- 2 455 097
- WO-A1-2007/094248
- WO-A1-2011/007885
- WO-A1-2012/099216
- WO-A1-2012/099224
- JP-A- 2007 531 509
- JP-A- 2011 503 170
- JP-A- 2011 512 345
- JP-A- 2011 524 750
- SASHINAMI H ET AL: "Salmon cartilage proteoglycan modulates cytokine responses to Escherichia coli in mouse macrophages", BIOCHEMICAL AND BIOPHYSICAL RESEARCH COMMUNICATIONS, ACADEMIC PRESS INC. ORLANDO, FL, US, vol. 351, no. 4, 29 December 2006 (2006-12-29), pages 1005-1010, XP024925791, ISSN: 0006-291X, DOI: 10.1016/J.BBRC.2006.10.146 [retrieved on 2006-12-29]
- MITSUI T ET AL: "Salmon cartilage proteoglycan suppresses mouse experimental colitis through induction of Foxp3<+> regulatory T cells", BIOCHEMICAL AND BIOPHYSICAL RESEARCH COMMUNICATIONS, ACADEMIC PRESS INC. ORLANDO, FL, US, vol. 402, no. 2, 12 November 2010 (2010-11-12), pages 209-215, XP027487711, ISSN: 0006-291X, DOI: 10.1016/J.BBRC.2010.09.123 [retrieved on 2010-10-20]
- TATSUJI TAKAHASHI ET AL: "Sake Binankotsu Proteoglycan no Kansetsutsu Oyobi Nankotsu Taisha ni Taisuru Yuyosei", FOOD STYLE 21, SHOKUHIN KAGAKU SHIMBUNSHA,FOOD CHEMICALS NEWSPAPER INC, JP, vol. 15, no. 10, 1 January 2011 (2011-01-01), pages 52-54, XP008175908, ISSN: 1343-9502
- Y. TATARA ET AL: "Epiphycan from salmon nasal cartilage is a novel type of large leucine-rich proteoglycan", GLYCOBIOLOGY, vol. 23, no. 8, 1 August 2013 (2013-08-01) , pages 993-1003, XP055218283, ISSN: 0959-6658, DOI: 10.1093/glycob/cwt038
- SAYURI YOSHIMURA ET AL: "Attenuation of Collagen-Induced Arthritis in Mice by Salmon Proteoglycan", BIOMED RESEARCH INTERNATIONAL, vol. 171, no. 11, 1 January 2014 (2014-01-01), pages 6173-9, XP055218272, ISSN: 2314-6133, DOI: 10.1002/eji.1830250508
- KAKIZAKI IKUKO ET AL: "Identification of proteoglycan from salmon nasal cartilage.", ARCHIVES OF BIOCHEMISTRY AND BIOPHYSICS 1 FEB 2011, vol. 506, no. 1, 1 February 2011 (2011-02-01), pages 58-65, XP55181897, ISSN: 1096-0384
- NUKA S ET AL: "Phenotypic characterization of epiphycan-deficient and epiphycan/biglycan double-deficient mice", OSTEOARTHRITIS AND CARTILAGE, BAILLIERE TINDALL, LONDON, GB, vol. 18, no. 1, 1 January 2010 (2010-01-01), pages 88-96, XP026812066, ISSN: 1063-4584 [retrieved on 2009-11-18]
- TATSUJI TAKAHASHI ET AL.: 'Sake Binankotsu Proteoglycan no Kansetsutsu Oyobi Nankotsu Taisha ni Taisuru Yuyosei' FOOD STYLE vol. 21, 15, no. 10, 2011, pages 52 - 54, XP008175908
- GOTO,M. ET AL.: 'Anti-aging effects of high molecular weight proteoglycan from salmon nasal cartilage in hairless mice' INT J MOL MED vol. 29, no. 5, May 2012, pages 761 - 8, XP055144959
- KAKIZAKI,I. ET AL.: 'Identification of proteoglycan from salmon nasal cartilage' ARCH BIOCHEM BIOPHYS vol. 506, no. 1, 2011, pages 58 - 65, XP055181897 & AKIRA SUGAWARA ET AL.: 'Sake Binankotsu Yurai Proteoglycan Natrium no Hinshitsu Hoji Gijutsu no Kaihatsu (Minkan To Kyodo Kenkyuhi)' HOKKAI DORITSU KUSHIRO SUISAN SHIKENJO JIGYO HOKOKUSHO vol. 2003, 2005, page 106

## Description

The present invention relates to a composition for use in preventing or treating osteoarthritis.

Osteoarthritis (OA) is a joint disease associated with chronic arthritis, and is a disease in which degeneration of articular components causes cartilage destruction and proliferative change of bone and cartilage. In particular, the number of knee osteoarthritis patients diagnosed through X-ray examination is approximately 25 million, and, of these patients, an estimated eight million or more experience pain.

In spite of these circumstances, only symptomatic treatments such as painkillers and direct intra-articular injection of hyaluronic acid are currently available for osteoarthritis. Arresting the development of osteoarthritis is virtually impossible. When the symptoms thereof become severe, surgery is the only method of treatment. Under such circumstances, there is a strong need for an effective therapeutic agent or therapeutic method.

In this context, EP 2 455 097 A1 describes usability of salmon nose cartilage proteoglycan in arthritic pain and cartilage metabolism. Further, Sashinami et al. (Sashinami, H. et al.; Biochemical and Biophysical Research Communications, 351; 2006; pp. 1005-1010) describes salmon cartilage proteoglycan modulating cytokine responses to Eschericia coli in mouse macrophages. Furthermore, Mitsui et al. (Mitsui, T. et al.; Biochemical and Biophysical Research Communications, 402; 2010; pp. 209-215) describes salmon cartilage proteoglycan suppressing mouse experimental colitis through induction of Foxp3+ regulatory T cells. Finally, Takahashi et al. (Takahashi, T. et al.; Food Style 21, Shokuhin Kagaku Shimbunsha, Food Chemicals Newspaper, 15(10); 2011; pp. 52-54) describes usability of salmon nasal cartilage proteoglycan in arthritic pain and cartilage metabolism.

Patent Literature 1: JP2007-262103A
Patent Literature 2: JP2009-274955A

An object of the present invention is to provide a means for preventing or treating osteoarthritis.

The present inventors unexpectedly discovered that high-molecular-weight proteoglycans are effective for preventing or treating osteoarthritis. The inventors have achieved the present invention with further improvements based on this finding.

Specifically, the present invention encompasses the inventions in the following items.
Item 1. A composition for use in preventing or treating osteoarthritis, comprising a fish cartilage water extract containing proteoglycans having molecular weights of not less than 5,000,000, wherein the amount of uronic acids derived from the proteoglycans having molecular weights of not less than 5,000,000 accounts for at least 30 mass% of the total uronic acid content of the fish cartilage water extract, and wherein the amount of uronic acids derived from the proteoglycans having molecular weights of not less than 1,800,000 accounts for at least 50 mass% of the total uronic acid content of the fish cartilage water extract, wherein the composition is for oral administration.
Item 2. The composition for use according to Item 1, wherein the fish cartilage water extract is a hot-water extract of fish cartilage.
Item 3. The composition for use according to Item 1 or Item 2, wherein the fish cartilage is salmon cartilage or trout cartilage.

The composition for use in preventing or treating osteoarthritis according to the present invention can prevent or treat osteoarthritis through oral intake.

The figures show:
Fig. 1 is a photograph of a frozen salmon nasal cartilage block. The lump disposed in the center of the plastic container is the frozen salmon nasal cartilage block.
Fig. 2 shows a uronic acid amount chromatogram and a 280-nm protein amount chromatogram of a fish cartilage water extract (freeze-dried product: Sample No. 1) containing high-molecular-weight proteoglycans.
Fig. 3 shows a uronic acid amount chromatogram and a 280-nm protein amount chromatogram of a commercially available proteoglycan product.
Fig. 4 shows an overview of the schedule for administering test samples to osteoarthritis model mice.
Fig. 5 shows Safranin O stained images of knee joints of osteoarthritis model mice to which test samples were administered.
Fig. 6a is a graph showing the analysis results of a "Safranin O" assay by the modified Mankin score in the osteoarthritis model mice to which the test samples were orally administered.
Fig. 6b is a graph showing the analysis results of a "Chondrocyte" (chondrocyte count) assay by the modified Mankin score in the osteoarthritis model mice to which the test samples were orally administered.
Fig. 6c is a graph showing the analysis results of a "Structure" (chondrocyte surface structure) assay by the modified Mankin score in the osteoarthritis model mice to which the test samples were orally administered.
Fig. 6d is a graph showing the analysis results of the sum of the scores of the "Safranin O," "Chondrocyte" (chondrocyte count), and "Structure" (chondrocyte surface structure) by the modified Mankin score in the osteoarthritis model mice to which the test samples were orally administered.

Proteoglycans are compounds containing glycosaminoglycans (mucopolysaccharides) linked to proteins. Glycosaminoglycans are acidic saccharides comprising repeating disaccharide units, and examples include chondroitin sulfate, dermatan sulfate, and heparan sulfate. In the repeating disaccharide units of these acidic saccharide components, one of the saccharides is an amino sugar, and the other is a uronic acid. Accordingly, the detection of proteoglycans can be performed by using the carbazole-sulfuric acid method, which is a known method for detecting uronic acids.

Further, compounds in which glycosaminoglycans are linked in a comb-like structure to proteins are referred to as proteoglycan monomers (the proteins in proteoglycan monomers are called core proteins). In particular, in a living body, such proteoglycan monomers are considered to bind to hyaluronic acids via link proteins to form aggregates. The aggregates are also called proteoglycan aggregates. The term "proteoglycan" used in this specification encompasses proteoglycan monomers and proteoglycan aggregates. Hyaluronic acid is a type of glycosaminoglycan.

The composition for use in preventing or treating osteoarthritis according to the present invention comprises a fish cartilage water extract containing high-molecular-weight proteoglycans.

The fish cartilage water extract contained in the composition for use in preventing or treating osteoarthritis described herein contains high-molecular-weight proteoglycans. The term "high-molecular-weight proteoglycans" used herein generally refers to proteoglycans having molecular weights of not less than 1,800,000, preferably molecular weights of not less than 2,500,000, not less than 3,000,000, not less than 4,000,000, not less than 5,000,000, not less than 6,000,000, not less than 7,000,000, not less than 8,000,000, not less than 9,000,000, not less than 10,000,000, not less than 11,000,000, not less than 12,000,000, not less than 13,00,000, not less than 14,000,000, not less than 15,000,000, not less than 16,000,000, not less than 17,000,000, not less than 18,000,000, not less than 19,000,000, or not less than 20,000,000. A greater molecular weight is preferable. Proteoglycans having molecular weights of not less than 5,000,000 are particularly preferable. The existence of proteoglycans having molecular weights of not less than a specific value as described above can be confirmed by subjecting the fish cartilage water extract to gel filtration chromatography under the following conditions, determining the amount of uronic acids (reflecting the amount of proteoglycans) in each fraction by using the carbazole-sulfuric acid method, and creating a chromatogram based on the determined uronic acid amount. This chromatogram based on the uronic acid amount may be hereinafter referred to as a "uronic acid amount chromatogram." Further, a chromatogram may also be created based on absorbance by measuring the absorbance of each fraction at 280 nm, and determining the relative protein amount based on the absorbance measurement results (i.e., the measured value is assumed to be a value that reflects the protein amount). This chromatogram may be hereinafter referred to as a "280 nm protein amount chromatogram."

### Gel Filtration Chromatography Conditions

Column: Sepharose CL-2B filled column (1-cm dia. × 50 cm column filled with Sepharose CL-2B as a carrier. Sepharose CL-2B has a dextran fractionation range of 100 to 20,000 kDa and is available from GE Healthcare and other companies. Sepharose CL-2B is a 2% crosslinked agarose with a particle size of 60 to 200 µm (measured by the laser diffraction scattering method), and is registered under CAS registry No. 65099-79-8.)
Buffer: 0.1 M phosphate buffer (pH 7.1, containing 0.2 M NaCl) Amount of applied sample: 4 mg of fish cartilage water extract (on a dry mass basis) (dissolved in 1 mL of buffer for use) Flow rate: 0.15 mL/min
Amount of fraction: 1 mL/tube
Molecular weight calibration curve: Various dextran molecular weight markers described below are subjected to gel filtration chromatography under the same conditions as described above. Absorbance (which reflects the dextran amount) of each fraction is measured by the phenol-sulfuric acid method, which is a well-known method for detecting saccharide, and the fraction in which each marker is eluted is determined to prepare a calibration curve reflecting the molecular weight of the components contained in each fraction in the gel filtration chromatography under the above conditions. The term "fraction in which each marker is eluted" refers to a fraction in which each marker is eluted most, i.e., a fraction corresponding to the peak maximum in a chromatogram reflecting the dextran amount when each dextran molecular weight marker is subjected to gel filtration.

### Dextran Molecular Weight Markers

| | |
|---|---|
| Dextran from *Leuconostoc mesenteroides* (mol wt 5,000,000 - 40,000,000) (Sigma) | for measuring the void volume of the column, 20,000k Da |
| Dextran Standard 1,400,000 (Sigma) | 1,400 kDa |
| Dextran Standard 670,000 (Sigma) | 670 kDa |
| Dextran Standard 410,000 (Sigma) | 410 kDa |
| Dextran Standard 270,000 (Sigma) | 270 kDa |

The dextran from *Leuconostoc mesenteroides* is used as a marker after being subjected to a pretreatment to remove low-molecular-weight dextran contained in the marker. The pretreatment is performed by eluting dextran from *Leuconostoc mesenteroides* under the conditions described above in "Gel Filtration Chromatography Conditions" to collect molecules having a molecular weight of not less than 20,000 kDa, and freeze-drying. More specifically, a chromatogram reflecting the dextran amount is prepared by measuring absorbance of each fraction by the phenol-sulfuric acid method, and the fraction corresponding to the first peak in the chromatogram is collected and freeze-dried (it is believed that molecules having a molecular weight of not less than 20,000 kDa are thereby collected and freeze-dried). This lyophilizate is actually used as a marker (for measuring the void volume of the column).

Measurement of absorbance to obtain a chromatogram reflecting the dextran amount is performed according to the method described in Hodge, J.E. and Hofreiter, B.T., Methods in Carbohydrate Chemistry, 1, 338 (1962) (the phenol-sulfuric acid method). More specifically, the measurement is carried out as follows.
[1] 500 µL of an aqueous solution of the sample is placed in a 105 × 15 mm test tube.
[2] 500 µL of a phenol reagent (5 v/v% aqueous phenol solution) is added thereto, and the mixture is stirred.
[3] 2.5 mL of concentrated sulfuric acid is added thereto, and the mixture is immediately stirred vigorously for 10 seconds.
[4] The mixture is left to stand for 20 minutes or more at room temperature.
[5] The absorbance at 490 nm is measured with a spectrophotometer.

The carbazole-sulfuric acid method refers to a well-known method comprising adding a carbazole solution, which is a dye for staining uronic acids (e.g., glucuronic acid (Glc A) and iduronic acid), to a measurement specimen, and measuring absorbance by using a spectrophotometer to determine uronic acid amount based on the absorbance. A calibration curve is prepared by using a glucuronic acid standard solution having a specific concentration, and the amount of glucuronic acid in the specimen is determined. More specifically, the carbazole-sulfuric acid method is performed in the following manner. 2.5 ml of a reagent obtained by dissolving 0.95 g of sodium borate decahydrate in 100 ml of concentrated sulfuric acid is placed in a test tube and ice-cooled. 0.5 ml of a test sample (preferably containing 2 to 20 µg of uronic acid) is gently layered thereon, and stirred well under ice-cooling so as to keep the resulting mixture lower than room temperature. After covering the test tube with a glass ball, heating is performed in a boiling-water bath for 10 minutes, followed by cooling with water to room temperature. A reagent obtained by dissolving 125 mg of carbazole in 100 ml of anhydrous methyl alcohol is added in an amount of 0.1 ml thereto and mixed. The mixture is further heated in a boiling-water bath for 15 minutes. Thereafter, the mixture is water-cooled to room temperature, and the absorbance at 530 nm is measured. As a blank, 0.5 ml of distilled water is used. Simultaneously, a calibration curve is prepared by using glucuronic acid. (The carbazole-sulfuric acid method in the Examples below is performed in the same manner as above.)

On a dry mass basis, at least 10 mass% of the uronic acids (determined by the carbazole-sulfuric acid method) contained in the fish cartilage water extract described herein are preferably derived from proteoglycans having molecular weights of not less than 1,800,000. In other words, the fish cartilage water extract is preferably such that the amount of uronic acids contained in proteoglycans having molecular weights of not less than 1,800,000 accounts for at least 10 mass% of the total uronic acid content of the extract, on a dry mass basis. The amount of uronic acids derived from proteoglycans having molecular weights of not less than 1,800,000 more preferably accounts for not less than 15 mass%, not less than 20 mass%, not less than 25 mass%, not less than 30 mass%, not less than 35 mass%, not less than 40 mass%, not less than 45 mass%, not less than 50 mass%, or not less than 55 mass%. A greater mass% is more desirable.

Further, the fish cartilage water extract described herein is preferably such that the amount of uronic acids contained in proteoglycans having molecular weights of not less than 2,500,000 accounts for at least 10 mass% of the total uronic acid content of the extract, on a dry mass basis. The amount of uronic acids derived from proteoglycans having molecular weights of not less than 2,500,000 more preferably accounts for not less than 15 mass%, not less than 20 mass%, not less than 25 mass%, not less than 30 mass%, not less than 35 mass%, not less than 40 mass%, not less than 45 mass%, not less than 50 mass%, not less than 55 mass%, or not less than 60 mass%. A greater mass% is more desirable.

Further, the fish cartilage water extract described herein is preferably such that the amount of uronic acids contained in proteoglycans having molecular weights of not less than 5,000,000 accounts for at least 10 mass% of the total uronic acid content of the extract, on a dry mass basis. The amount of uronic acids derived from proteoglycans having molecular weights of not less than 5,000,000 more preferably accounts for not less than 10 mass%, not less than 13 mass%, not less than 16 mass%, not less than 20 mass%, not less than 24 mass%, not less than 27 mass%, not less than 30 mass%, not less than 34 mass%, or not less than 37 mass%. A greater mass% is more desirable.

The proportion of the uronic acid amount of proteoglycans having molecular weights of not less than a specific value (expressed as X hereinbelow) relative to the total uronic acid content of the extract can be determined from the peak area in the above-mentioned uronic acid amount chromatogram. More specifically, the proportion can be determined by calculating the proportion of the area of uronic acids having a molecular weight of not less than X relative to the entire peak area in the uronic acid amount chromatogram. Even more specifically, the proportion can be determined in the following manner. On a uronic acid amount chromatogram in which uronic acid amount is plotted on the ordinate versus fraction No. on the abscissa, a vertical line is drawn so that the vertical line passes through the fraction containing the proteoglycan having a molecular weight of X. Of the two peak portions divided by the vertical line, the proportion of the area of the peak portion containing proteoglycans having greater molecular weights relative to the entire peak area is calculated.

The uronic acids contained in the fish cartilage water extract described herein may include those contained in sugar chains cleaved from proteoglycans as well as those contained in proteoglycans.

Further, the uronic acid content (measured by the carbazole-sulfuric acid method) of the fish cartilage water extract described herein is, on a dry mass basis, preferably not less than 5 mass% of the extract, more preferably not less than 7.5 mass%, even more preferably not less than 10 mass%, still more preferably not less than 12.5 mass%, still even more preferably not less than 15 mass%, and particularly preferably not less than 17.5 mass%. When the uronic acid amount or content is expressed in the present specification (in particular, in figures and tables), the term "GlcA," for example, which is an abbreviation for glucuronic acid, may be used and indicated as "GlcA (µg)." Most of the glycosaminoglycans of proteoglycans contained in the fish cartilage water extract are considered to be chondroitin sulfate. It is known that an approximate amount of chondroitin sulfate can be obtained by multiplying the uronic acid amount by a conversion factor of 2.593. Accordingly, an approximate amount of proteoglycans contained in the fish cartilage water extract described herein can be calculated by multiplying the uronic acid amount by a conversion factor of 2.593.

The fish cartilage water extract described herein is extracted from fish cartilage (cartilage of fishes). The type of fish is preferably *Oncorhynchus* (*Salmonidae*), including trout (humpback salmon, cherry salmon, satsukimasu salmon, etc.), salmon (chum salmon, sockeye salmon, silver salmon, Chinook salmon, steelhead, etc.), shark, and cod. Salmon and trout are particularly preferable. The cartilage to be used is not particularly limited; however, head cartilage, in particular nasal cartilage, is preferable. Moreover, since fish (in particular, salmon and trout) heads are usually discarded when fish are processed into food products, the cost of fish heads is low, and a large amount of fish heads can be stably supplied, which is another advantage.

The extraction is performed using water. Fish cartilage obtained from a biological sample may be directly subjected to extraction, or after being pulverized (more specifically, fragmented or powdered). As described below, fish cartilage may be defatted using an organic solvent such as ethanol before the extraction. As such, proteoglycans (including high-molecular-weight proteoglycans) can be extracted using water. Alternatively, water extraction may be performed while heating water or by using hot or boiling water, whereby a fish cartilage water extract with a higher effect can be efficiently obtained.

As described above, as fish cartilage, cartilage obtained from a biological sample can be directly subjected to extraction. However, the fish cartilage is preferably kept frozen until subjected to extraction. The freezing method is not particularly limited, and any known freezing method can be used. For example, a method of storing fish cartilage in a freezer at about -20 to -80°C for about 24 to 72 hours can be used. Defatted fish cartilage (i.e., fish cartilage from which lipids are removed) may also be used. The use of defatted fish cartilage is advantageous in that a highly purified fish cartilage water extract that contains fewer lipids can be obtained. Examples of the defatting method include the method for obtaining "defatted fish cartilage" described below.

Small fish cartilage pieces are obtained by fragmenting fish cartilage into small pieces. The fragmentation may be performed by using a known method. For example, fish cartilage (preferably frozen fish cartilage) may be fragmented into small pieces by using a known device, such as a blender or a mill. The fragmenting operation is preferably performed at a low temperature. For example, the fragmentation is preferably performed at a temperature at which the fragmented fish cartilage can be kept frozen. More specifically, the fragmentation is preferably performed at a temperature of 0°C or lower.

Further, in terms of extraction efficiency, the small fish cartilage pieces are preferably frozen small pieces of fish cartilage (small frozen fish cartilage pieces). The small frozen fish cartilage pieces can be obtained by (i) freezing fish cartilage, and then fragmenting the frozen fish cartilage into small pieces, or by (ii) fragmenting fish cartilage into small pieces, and then freezing the small fish cartilage pieces. The small frozen fish cartilage pieces obtained by method (i) are more preferable. The freezing method is not particularly limited, and any known freezing method can be used. For example, a method of storing fish cartilage in a freezer at about -20 to -80°C for about 24 to 72 hours can be used.

The small fish cartilage pieces or small frozen fish cartilage pieces preferably weigh about 0.001 to 0.5 g, more preferably about 0.005 to 0.3 g, and even more preferably about 0.01 to 0.1 g per piece. The fragmenting operation is preferably performed in a manner enabling the production of such small pieces (the conditions for obtaining such small pieces can be easily obtained by appropriately selecting and adjusting the device to be used).

Fish cartilage powder is obtained by pulverizing fish cartilage into powder (powdered fish cartilage). The pulverization may be performed by using a known method. For example, the pulverization of fish cartilage (preferably frozen fish cartilage) may be performed by using a known device, such as a blender or a mill. The pulverization is preferably performed at a low temperature (e.g., not more than 0°C).

Further, in terms of extraction efficiency, it is preferable to use frozen powder of fish cartilage (frozen fish cartilage powder). Frozen fish cartilage powder can be obtained by (i') freezing fish cartilage, and then pulverizing the frozen fish cartilage into powder, or by (ii') pulverizing fish cartilage into powder, and then freezing the powder. The frozen fish cartilage powder obtained by method (i') is more preferable. The freezing method is not particularly limited, and any known freezing method can be used. For example, a method of storing fish cartilage in a freezer at about -20 to -80°C for about 24 to 72 hours can be used.

The term "powder" refers to a piece smaller than what is referred to by the term "small pieces"; however, these terms are not necessarily distinguished clearly. Among the products resulting from pulverization of fish cartilage, relatively large pieces are referred to as "small pieces," and relatively small pieces are referred to as "powder." Therefore, although there is no particular limitation, the powder preferably contains particles having a particle size of about 10 to 1,000 µm, preferably about 50 to 500 µm, more preferably about 100 to 200 µm (measured by a laser diffraction and scattering method). The powder preferably contains particles having the above particle size in a large proportion (e.g., not less than 50 mass%, preferably not less than 70 mass%).

As the small fish cartilage pieces or fish cartilage powder, defatted fish cartilage (i.e., fish cartilage pieces or fish cartilage powder from which lipids are removed) may also be used. In other words, defatted small fish cartilage pieces or defatted fish cartilage powder may also be used. By using defatted fish cartilage, a highly purified fish cartilage water extract that contains fewer lipids can be obtained. The defatted small fish cartilage pieces or defatted fish cartilage powder can be obtained by (α) pulverizing defatted fish cartilage into small pieces or powder, or (β) pulverizing fish cartilage into small pieces or powder, and then defatting the small pieces or powder.

The defatting may be performed by a known method. For example, fish cartilage defatting step (α) above may be performed by placing fish cartilage under running water (e.g., tap water) for about 1 to 24 hours. Preparation of fish cartilage can be performed using a known method, such as a method comprising immersing fish tissues (preferably a fish head) in water for about 1 to 24 hours to swell the tissues, and removing tissues other than cartilage (preferably nasal cartilage), and a method comprising thawing a frozen salmon head, then immediately separating the nasal cartilage, and placing the nasal cartilage under running water for about 1 to 24 hours, thereby washing and defatting the cartilage. When the cartilage has residual flesh, the flesh is preferably removed with tweezers or the like. At this point, since the fish cartilage has not been pulverized into small pieces or powder, little proteoglycan is likely to be extracted, even if the cartilage is placed under running water. Further, as in step (β) below, lipids can also be removed by extraction using an organic solvent.

The step (β) of defatting the small fish cartilage pieces or fish cartilage powder may be performed, for example, by a method comprising extracting and removing lipids using an organic solvent. Examples of the organic solvent include ethanol, hexane, and acetone. More specifically, as step (β), a method disclosed in JP2009-173702A can be preferably used. More specifically, for example, defatted fish cartilage powder, which is obtained by a method including the following steps A to E, can be preferably used (JP2009-173702A also discloses more detailed conditions).
A. Frozen aquatic animal tissues (fish tissues) are crushed, and treated at 0 to 20°C, pH of 4.8 to 7 after adding water.
B. A solid-liquid mixture obtained by step A is centrifuged to remove the uppermost lipid layer and the intermediate aqueous layer to collect a precipitate.
C. The precipitate is dried and pulverized into a fine powder.
D. A solvent, namely hexane, acetone, or ethanol, is added to the obtained dried fine powder to extract and remove residual lipids.
E. The solvent is removed.

It is more preferable to use frozen and defatted small fish cartilage pieces or fish cartilage powder (frozen, defatted small fish cartilage pieces or frozen, defatted fish cartilage powder). These can be obtained, for example, by freezing defatted fish cartilage, and pulverizing the frozen fish cartilage into small pieces or powder.

These defatting methods can be applied not only to small fish cartilage pieces or fish cartilage powder, but also to cartilage obtained from a biological sample.

The fish cartilage (including small fish cartilage pieces and fish cartilage powder, which hereinafter may be referred to collectively as "fine fish cartilage") is subjected to water extraction. Examples of water used for water extraction (which hereinafter may be referred to as "extraction water") include Milli-Q water, distilled water, deionized water, purified water, and tap water. Further, the pH of the extraction water is typically about 5.5 to 8.0, preferably about 6.0 to 7.5, more preferably about 6.5 to 7.5. It is not preferable to dissolve substances that greatly change the pH, such as acids, alkalis, and bases. If an acid compound such as an organic acid or an inorganic acid, or an alkali compound such as sodium hydroxide, is added to the extraction water, high-molecular-weight proteoglycans (in particular, high-molecular-weight proteoglycans having molecular weights of higher than 10,000,000) are reduced or disappear. Accordingly, no addition of acid compounds or alkali compounds is preferable. Although a restrictive interpretation is not desired, this presumably occurs because acid compounds and alkali compounds cause degradation of proteoglycan aggregates during extraction.

The water extract can be obtained, for example, by immersing fish cartilage in water for an appropriate period of time (e.g., not less than 30 minutes, preferably about 30 minutes to 24 hours, more preferably about 1 to 12 hours, even more preferably about 2 to 6 hours, and still more preferably about 3 to 4 hours). The amount of water is not particularly limited; for example, the water amount is a sufficient amount for completely immersing all of the small fish cartilage pieces or fish cartilage powder subjected to extraction. The water extraction may be performed while allowing to stand or while stirring. Stirring is preferable. The water temperature during the extraction is not particularly limited; however, the temperature is preferably not less than 50°C, and more preferably not less than 70°C. To ensure this temperature range, the water may be heated during the extraction or before the extraction. The heating temperature (i.e., the temperature of the water used) is preferably about 50 to 100°C, more preferably about 70 to 100°C, even more preferably about 80 to 100°C, still more preferably about 90 to 100°C. The heating may be performed under an increased pressure. Since heating may cause degradation of high-molecular-weight proteoglycans, the heated extraction water may be replaced during the extraction. The extraction water may be replaced, for example, every 15 minutes to 4 hours, preferably every 30 minutes to 2 hours, or approximately every 1 hour. A preferable embodiment of water extraction is, for example, a method comprising adding water (preferably heated water) to fish cartilage in an amount sufficient to completely immerse the total amount of fish cartilage, and allowing it to stand or stirring it for 3 to 4 hours while heating. Another preferable embodiment is a method comprising repeating the following process four times: adding water (preferably heated water) to fish cartilage in an amount sufficient to completely immerse the total amount of fish cartilage, allowing it to stand for an hour while heating, and collecting the resulting water (in this case, the water extraction is performed for 4 hours in total).

After the water extraction, the liquid portion is collected to obtain a fish cartilage water extract. The collection of the liquid portion can be performed, for example, by collecting the supernatant through a centrifugation treatment (e.g., centrifugation at 5000 rpm, 4°C, for 20 minutes) or a continuous centrifugation treatment. The liquid (supernatant) may be used unmodified as the fish cartilage water extract, or may be further purified by a known method (e.g., defatting). Alternatively, the liquid may be concentrated by distillation under reduced pressure or the like. It is also possible to dry or powder the liquid according to the freeze-drying method or the spray-drying method.

For example, the thus-obtained fish cartilage water extract containing high-molecular-weight proteoglycans is preferably used as a composition for preventing or treating osteoarthritis.

The composition for use in preventing or treating osteoarthritis according to the present invention comprises a fish cartilage water extract containing high-molecular-weight proteoglycans. The composition for use in preventing or treating osteoarthritis of the present invention is preferably used in the pharmaceutical and food fields.

When the composition for use in preventing or treating osteoarthritis is used in the pharmaceutical field, the composition (hereinafter, sometimes referred to as the "pharmaceutical composition") may consist only of a fish cartilage water extract containing high-molecular-weight proteoglycans, or may contain other components. The pharmaceutical composition may contain pharmaceutically acceptable bases, carriers, and additives (e.g., excipients, binders, disintegrators, lubricants, solvents, sweeteners, colorants, corrigents, odor-masking agents, surfactants, moisturizers, preservatives, pH adjusters, and thickeners). Such bases, carriers, additives, etc., are specifically described, for example, in Japanese Pharmaceutical Excipients Directory 2007 (Yakuji Nippo Limited), and those mentioned therein, for example, may be used. The composition can be formed into a preparation form, such as tablets, coated tablets, powders, granules, fine granules, capsules, pills, liquids, suspensions, emulsions, jellies, chewables, or soft tablets, by using a known method.

The amount of the high-molecular-weight proteoglycan-containing fish cartilage water extract in the pharmaceutical composition described herein is not particularly limited as long as effects of preventing or treating osteoarthritis are provided. The amount can be suitably set according to the preferred daily intake amount of the fish cartilage water extract. The amount is preferably 0.0005 to 100 mass%, more preferably 0.005 to 90 mass%, and even more preferably 0.05 to 80 mass%.

The subject to whom the pharmaceutical composition described herein is to be administered is an osteoarthritis patient. In particular, a gonarthrosis patient is preferable. The severity of illness of the patient is not particularly limited, and the composition can be administered to early-stage patients, intermediate-stage patients, and late-stage patients. It is also possible to preventatively use the composition for people having a high risk of developing osteoarthritis, such as elderly people.

The timing of administering the pharmaceutical composition is not particularly limited, and can be appropriately selected by taking into consideration, for example, the dosage form, patient's age, severity of patient's symptoms, etc. The mode of administration is preferably oral administration. When the target subject is a patient to whom the pharmaceutical composition is hard to administer via an oral route, such as a patient with dysphagia, the composition may be fed directly to the stomach through a gastrostomy tube.

The dosage of the pharmaceutical composition can be suitably selected according to the patient's age, severity of patient's symptoms, and other conditions. The amount of high-molecular-weight proteoglycans in the pharmaceutical composition is preferably set so that the daily intake amount per adult is within the range of 1 to 1,000 mg, and more preferably 10 to 300 mg. The pharmaceutical composition can be administered once per day, or administered in separate doses several times (preferably, 2 to 3 times) per day.

When the composition for use in preventing osteoarthritis is used as a food additive, the composition (hereinafter sometimes referred to as the "food additive") may consist only of a fish cartilage water extract containing high-molecular-weight proteoglycans, or may comprise the fish cartilage water extract and other components such as food-hygienically acceptable bases, carriers, additives, and other components and materials that can be used as food additives. Examples of the forms of such food additives include, but are not limited to, liquids, powders, flakes, granules, and pastes. Specific examples of food additives include seasonings (e.g., soy sauce, Worcestershire sauce, ketchup, and dressing), flakes (*furikake* [seasoning mix for sprinkling over cooked rice]), *yakiniku* [Korean-style barbecue] sauce, spices, and paste-like roux (e.g., paste-like curry roux). These food additives can be appropriately prepared according to known methods. The amount of the high-molecular-weight proteoglycan-containing fish cartilage water extract in the food additive described herein is not particularly limited, as long as effects of preventing or treating osteoarthritis are provided. The amount is preferably 0.0005 to 100 mass%, more preferably 0.005 to 90 mass%, and even more preferably 0.05 to 80 mass%.

Eating a food comprising such a food additive results in intake of the food additive described herein. The food additive may be added to a food while the food is cooked or produced, or may be added immediately before or while the cooked food is eaten. Oral intake of the food additive in this manner provides an osteoarthritis-preventive effect. Various conditions, such as the subject receiving the food additive, and the intake amount of high-molecular-weight proteoglycans contained in the food additive, are not particularly limited, but are preferably, for example, the same as those described above for the pharmaceutical composition described herein.

When the composition for use preventing osteoarthritis is used as a food or beverage, the composition (hereinafter sometimes referred to as the "food or beverage") comprises the fish cartilage water extract and other components, such as food-hygienically acceptable bases, carriers, additives, and other ingredients and materials that can be used for foods. Examples includes foods and beverages that comprise a fish cartilage water extract containing high-molecular-weight proteoglycans, such as processed foods, beverages, health foods (e.g., foods with nutrient function claims and foods for specified health uses), supplements, medical foods (e.g., hospital diets, sick diets, and nursing-care foods), and the like. Examples further include those produced by forming the high-molecular-weight proteoglycan-containing fish cartilage water extract into a powder by freeze-drying or spray-drying, and incorporating the powder into various beverages and foods, such as beverages (e.g., juices), confectionaries (e.g., gums, gummy candies, chocolates, biscuits, cookies, *okaki* (rice crackers), *sembei* (rice crackers), rice crackers, puddings, jellies, and *annin tofu* (almond jelly)), breads, soups (including powdered soups), and processed foods.

When the food or beverage described herein is prepared as health foods (e.g., food with nutrient function claims, and food for specified health use) or supplements, preferable forms thereof are granules, capsules, pills (including, for example, chewable tablets), and beverages (drink preparations) in view of ease of continuous intake. Among these, in terms of ease of intake, forms such as capsules, tablets, and pills are preferable, but are not particularly limited thereto. The food or beverage in the form of granules, capsules, pills, or the like, can be appropriately prepared according to known methods using pharmaceutically and/or food-hygienically acceptable carriers or the like. When the food or beverage is formed into other forms, known methods may also be used.

The amount of proteoglycan-containing fish cartilage water extract in the food or beverage described herein is not particularly limited as long as an effect of preventing osteoarthritis is provided. The amount is preferably 0.0005 to 100 mass%, more preferably 0.005 to 90 mass%, and still more preferably 0.05 to 80 mass%.

The food or beverage described herein is preferably used for preventing osteoarthritis. Various conditions, such as the subject receiving the food or beverage, and the intake amount of high-molecular-weight proteoglycans contained in the food or beverage, are not particularly limited, but are preferably the same as, for example, those described above for the pharmaceutical composition described herein.

Hospital diets are meals provided to hospitalized people. Sick diets are meals for the sick. Nursing-care foods are meals for people receiving care. The food or beverage described herein is particularly preferably used as hospital diets, sick diets, or nursing-care foods that are for patients hospitalized due to osteoarthritis, patients recuperating therefrom at home, or patients receiving nursing care. People having a high risk of developing osteoarthritis, such as elderly people, may also preventatively ingest the food or beverage.

Further described herein is a method for preventing or treating osteoarthritis, comprising orally administering or ingesting the composition for use in preventing or treating osteoarthritis described herein to osteoarthritis patients, or persons having a high risk of developing osteoarthritis. Specifically, these methods can be performed through oral administration or oral intake of the composition for use in preventing or treating osteoarthritis described herein. In this method, each of the conditions, such as oral administration and intake amount, are as described above.

### Examples

The present invention is more specifically described below. However, the present invention is not limited to the following examples.

### Preparation of Proteoglycans

A proteoglycan-containing water extract was obtained from salmon nasal cartilage in the following manner. Salmon nasal cartilage was obtained by separating nasal cartilage immediately after thawing a frozen salmon head, placing the nasal cartilage under running water for 6 hours to wash and defat the nasal cartilage, removing pieces of flesh and the like with tweezers, and washing the nasal cartilage with water by hand.

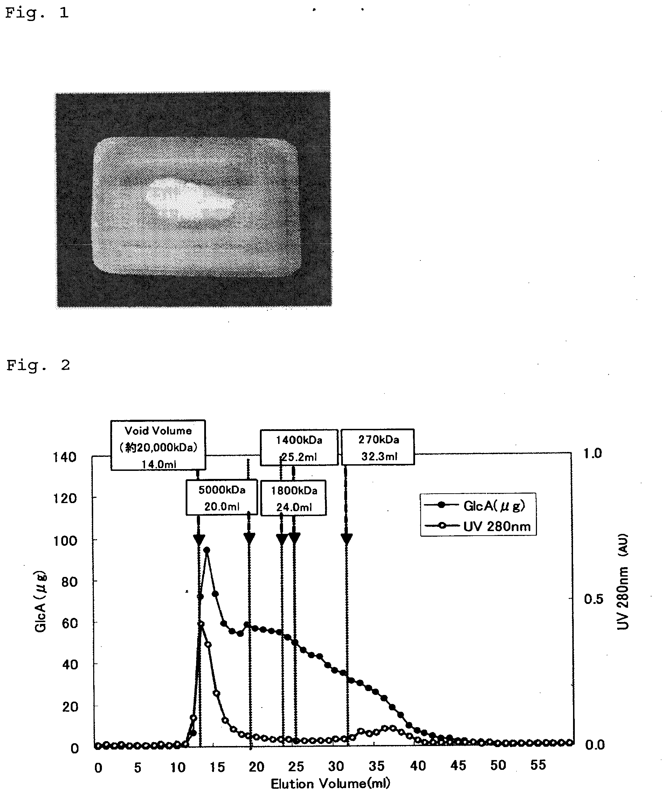

The salmon nasal cartilage was stored and frozen in a freezer, and the frozen nasal cartilage was used as a "frozen salmon nasal cartilage block." Fig. 1 is a photograph of the frozen salmon nasal cartilage block. The frozen salmon nasal cartilage block had a size of about 2.5 × 1.5 cm to about 4.5 × 2 cm, and a weight of about 1.71 g to about 6.91 g (the average weight of 7 blocks was 3.701 g); however, the size and weight depend on the size of the salmon head used.

Proteoglycans were extracted by heating a frozen salmon nasal cartilage block at 100°C. Specifically, the extraction was performed in the following manner. 2,500 mL of distilled water was added to a total of about 1,000 g of the frozen salmon nasal cartilage blocks, and the resulting mixture was heated at 100°C for 3 hours. The mixture was centrifuged with a centrifugal separator at 8,000 rpm at 4°C for 30 minutes to remove insoluble matter (residue) and collect the supernatant. The collected supernatant was suction-filtered using filter paper. The obtained filtrate was freeze-dried to obtain a proteoglycan-containing lyophilizate. The lyophilizate was crushed with a cutter mill, pulverized into powder, and subjected to the following analysis. About 65 g of powder was obtained. The proteoglycan-containing lyophilized powder is referred to as "Sample No. 1."

### Analysis of Molecular Weight

Sample No. 1 was separated into fractions by gel filtration chromatography under the conditions described below. The amount of uronic acids contained in each fraction was quantified by the carbazole-sulfuric acid method. In addition, absorbance at 280 nm of each fraction was measured, and the absorbance was defined as a value reflecting the amount of protein contained therein. Based on these results, a uronic acid amount chromatogram and a 280-nm protein amount chromatogram were drawn. Fig. 2 shows a superimposition of the uronic acid amount chromatogram and the 280-nm protein amount chromatogram. The amount of uronic acid in the total amount of Sample No. 1 (about 65 g) was about 12 g.

Fig. 2 shows the uronic acid amount chromatogram together with the position of each fraction in which each molecular weight marker was eluted. Since the amount of each fraction in the gel filtration chromatography was 1 mL/tube as described below, the horizontal axis, i.e., "Elution Volume (mL)," in Fig. 2 also reflects the fraction No.

### Gel Filtration Chromatography Conditions

Column: Sepharose CL-2B filled column (1-cm dia. × 50 cm column filled with Sepharose CL-2B as a carrier.
Sepharose CL-2B has a dextran fractionation range of 100 to 20,000 kDa, and is available from GE Healthcare and other companies.
Sepharose CL-2B is a 2% crosslinked agarose with a particle size of 60 to 200 µm (measured by the laser diffraction scattering method), and is registered under CAS registry No. 65099-79-8.) Buffer: 0.1 M phosphate buffer (pH of 7.1, containing 0.2 M NaCl)
Amount of applied sample: 1 mg/ml in terms of uronic acid
Flow rate: 0.15 mL/min
Amount of fraction: 1 mL/tube
Molecular weight analytical curve: The various dextran molecular weight markers described below were subjected to gel filtration chromatography under the same conditions as described above (except that the amount of the sample applied was 1 mg/1 mL of buffer), and absorbance (which reflects the dextran amount) of each fraction was measured by the phenol-sulfuric acid method to prepare a calibration curve.

### Dextran Molecular Weight Markers

| | |
|---|---|
| Dextran from *Leuconostoc mesenteroides*(mol wt 5,000,000 - 40,000,000) (Sigma) | for measuring the void volume of the column, 20,000k Da |
| Dextran Standard 1,400,000 (Sigma) | 1,400 kDa |
| Dextran Standard 670,000 (Sigma) | 670 kDa |
| Dextran Standard 410,000 (Sigma) | 410 kDa |
| Dextran Standard 270,000 (Sigma) | 270 kDa |

The dextran from *Leuconostoc mesenteroides* was used after being subjected to a pretreatment to remove low-molecular-weight dextran contained in the marker. The pretreatment was performed by eluting the dextran from *Leuconostoc mesenteroides* under the conditions described above in "Gel Filtration Chromatography Conditions" (the applied amount was the amount for marker) to collect molecules having a molecular weight of not less than 20,000 kDa, and freeze-drying. More specifically, a chromatogram reflecting the dextran amount was prepared by measuring the absorbance of each fraction by the phenol-sulfuric acid method. The fraction that corresponded to a first peak in the chromatogram was collected and freeze-dried (it is believed that dextran having a molecular weight of not less than 20,000 kDa was thereby obtained). This lyophilizate was actually used as a marker (for measuring the void volume of the column).

Measurement of absorbance to obtain a chromatogram reflecting the dextran amount was performed according to the method (the phenol-sulfuric acid method) described in Hodge, J.E. and Hofreiter, B.T., Methods in Carbohydrate Chemistry, 1, 338 (1962). More specifically, the measurement was carried out as follows.
[1] 500 µL of a sample aqueous solution is placed in a 105 × 15 mm test tube.
[2] 500 µL of a phenol reagent (5 v/v% aqueous phenol solution) is added thereto, and the mixture is stirred.
[3] 2.5 mL of concentrated sulfuric acid is added thereto, and the mixture is immediately stirred vigorously for 10 seconds.
[4] The mixture is left to stand for 20 minutes or more at room temperature.
[5] The absorbance at 490 nm is measured with a spectrophotometer.

The obtained calibration curve was (y = -4.3446Ln(x) + 56.68; R² = 0.9823). From the R² value, it was found that the molecular weight and the fraction No. (i.e., elution volume) were highly correlated.

As shown in Fig. 2, Sample No. 1 was found to contain high-molecular-weight proteoglycans having molecular weights of not less than 1,800,000 (in particular, a molecular weight of not less than 5,000,000).

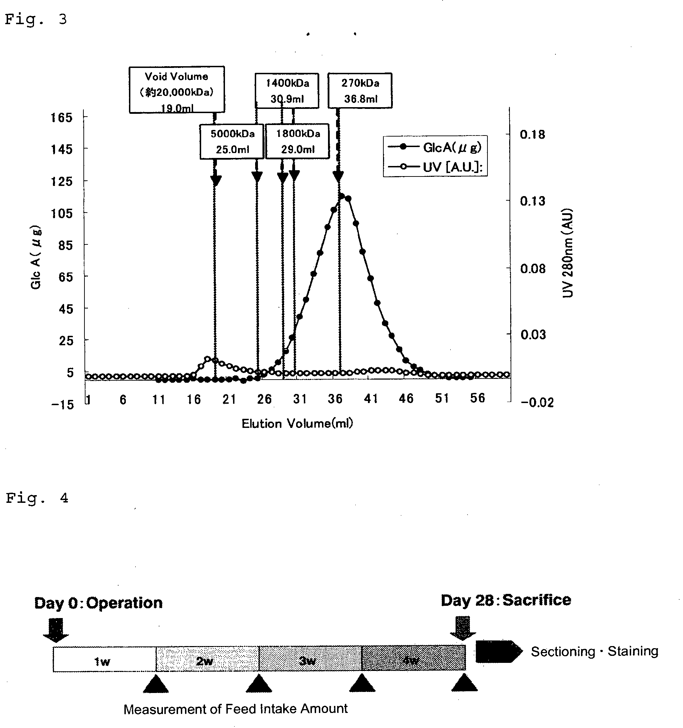

A commercially available "proteoglycan" product was also analyzed in the same manner for comparison. Fig. 3 shows a superimposition of a uronic acid amount chromatogram and a 280-nm protein amount chromatogram. In the uronic acid amount chromatogram of Fig. 3, the position of each fraction at which each dextran molecular weight marker was eluted was also shown. The obtained calibration curve was (y = -3.943Ln(x) + 59.069; R² = 0.9978). From the R² value, it was found that the molecular weight and the fraction No. (i.e., elution volume) were highly correlated. As shown in Fig. 3, the commercially available proteoglycan product substantially contains no proteoglycans having molecular weights of not less than 1,800,000 and in particular, contains no proteoglycans having molecular weights of not less than 5,000,000. The commercially available proteoglycan product is hereinafter referred to as "Sample No. 2."

The proportion of the uronic acid amount of proteoglycans having molecular weights of not less than 1,800,000 in each of Sample Nos. 1 and 2, relative to the total uronic acid content of the entire sample, was calculated based on the uronic acid chromatogram shown in Fig. 2 or 3. More specifically, the proportion was obtained by calculating the proportion of the area of uronic acids having molecular weights of not less than 1,800,000 based on the entire peak area in the uronic acid amount chromatogram shown in Fig. 2 or 3. Even more specifically, on the uronic acid amount chromatogram, a vertical line was drawn from an elution volume point corresponding to a molecular weight of 1,800,000 in the uronic acid amount chromatogram, and calculating the ratio of the two areas of the chromatogram divided by the vertical line. The proportion of the uronic acid amount of proteoglycans having molecular weights of not less than 5,000,000 in each of Sample Nos. 1 and 2, relative to the uronic acid content of the entire sample, was also calculated in a similar manner. Table 1 shows the results.

**Table 1**

| Molecular weight | Not less than 5,000,000 (500 × 10⁴) | Not less than 1,800,000 (180 × 10⁴) |
|---|---|---|
| Sample No. 1 | 37.9% | 55.8% |
| Sample No. 2 | 0.0% | 3.0% |

### Examination of the Effect on Osteoarthritis

### <Production of model mice>

Osteoarthritis model mice were produced in the following manner. Six-week-old C57bl6 mice (males: about 20 to 22 g) were purchased from Japan SLC, Inc. 0.3 ml of Ketalar (50 mg/ml) and 0.1 ml of Celactal (2%) were subcutaneously injected into a thigh of each of the mice to place the mice under general anesthesia. The mice were shaved around the knee joints and prepared for surgery. The right hind paw of the mice was subjected to anterior cruciate ligament transection and medial meniscectomy. Conversely, in the left hind paw of the mice, the joint capsule was incised similarly to the right paw; however, the skin was sutured without damaging the ligament or meniscus to perform a sham operation (sham surgery). Moderately impaired mice that were subjected to anterior cruciate ligament transection and partial meniscectomy were thus prepared.

### Administration of Test Samples to the Model Mice

The mice were divided into four groups as shown in Table 2 (n = 10). With the assumption that the daily intake amount of feed per mouse is 4 g, each sample (Sample No. 1 or 2) was added to a general feed for experimental animals in an amount shown in Table 2 to make the total amount 4 g. However, since Sample No. 2 contained an excipient, Sample No. 2 was added in an amount such that the proteoglycan content of the feed was 5 mg (Table 2).

**Table 2**

| Group | Content |
|---|---|
| | Feed sample |
| Group 1 | 0.5 mg of Sample No. 1, 4 g of feed per day |
| Group 2 | 5 mg of Sample No. 1, 4 g of feed per day |
| Group 3 | 25 mg of Sample No. 1, 4 g of feed per day |
| Group 4 | 5 mg of Sample No. 2, 4 g of feed per day |

After performing surgery to prepare the above model mice, a test was conducted according to the following schedule. Specifically, the feeds containing the samples were fed to the model mice for 4 weeks after the surgery. During the feeding period, the mice were kept, 5 mice per cage, at a room temperature of 23±2°C and a humidity of 50-60%, with free access to feed; activity was not restricted. The intake amount was measured when the feeds were replaced every one week; the daily intake amount per mouse was estimated. Fig. 4 shows an overview of the test schedule. A control group that received only a general feed for experimental animals (CE-2) was also investigated.

Four weeks after the surgery, each mouse was placed under anesthesia (0.3 ml of Ketalar (50 mg/ml) and 0.1 ml of Celactal (2%) were subcutaneously injected) to collect blood from the heart and remove the knee joints. After the mice were shaved in the same manner as for surgery, each femur and tibia pair were cut off, placed in the same direction, placed in a sample pack, and fixed in 4% paraformaldehyde for 24 hours. Thereafter, the bones were decalcified with EDTA (0.5 mol) for 3 weeks, and the resulting bone tissues were embedded in paraffin to prepare decalcified specimens having a thickness of about 5 µm. The specimens were sectioned and then stained with hematoxylin and eosin (HE staining), and with Safranin O (Safranin O staining). Safranin O is a basic dye that binds to acidic glycosaminoglycans to produce an orange color. Therefore, Safranin O is used as an indicator of cartilage tissue. After staining, three evaluators scored each group in terms of the following three items: "Safranin O" (staining range; indicating the amount of glycosaminoglycans), "Chondrocyte" (the number of chondrocytes), and "Structure" (cartilage surface structure) using the modified Mankin score, and evaluated joint cartilage lesion using the average values. Statistical analysis was performed according to the Bonferroni/Dunn method for multiple comparisons.

The Mankin score is highly reliable because 1) comparisons are made with human cases, and 2) changes are examined over time. The Mankin score is generally used as a method for evaluating cartilage degeneration. Table 3 shows the criteria of each item of the modified Mankin score used in this analysis.

**[Table 3]**

| **Table 3 Modified Mankin score (criteria for histological evaluation)** |
|---|
| Safranin O-fast green staining |
| 0 = uniform staining throughout articular cartilage |
| 1 = loss of staining in die superficial zone for less than one-half of the length of the plateau |
| 2 = loss of staining in the superficial zone for one-half or more of the length of the plateau |
| 3 = loss of staining in the superficial and middle zones for less than one-half of the length of the plateau |
| 4 = loss of staining in the superficial and middle zones for one-half or more of the length of the plateau |
| 5 = loss of staining in all 3 zones for less than one-half of the length of the plateau |
| 6 = loss of staining in all 3 zones for one-half or more of the length of the plateau |
| |

| Chondrocyte loss |
|---|
| 0 = no decrease in cells |
| 1 = minimal decrease in cells |
| 2 = moderate decrease in cells |
| 3 = marked decrease in cells |
| 4 = very extensive decrease in cells |
| |

| Structure |
|---|
| 0 = normal |
| 1 = surface irregularities |
| 2 = 1-3 superficial clefts |
| 3 = >3 superficial clefts |
| 4 = 1-3 clefts extending into the middle zone |
| 5 = >3 clefts extending into the middle zone |
| 6 = 1-3clefts extending into the deep zone |
| 7 = >3 clefts extending into the deep zone |
| 8 = clefts extending to calcified cartilage |

### <Analysis results>

Table 4 shows the measurement results of the feed intake amount and body weight (average values) of each group.

**Table 4**

| | Intake amount (g/day) | Body weight (g) immediately after the surgery | Body weight (g) four weeks after the surgery |
|---|---|---|---|
| Control | 3.6 | Not recorded | Not recorded |
| Group 1 | 5.18 | 22.59 | 25.59 |
| Group 2 | 4.93 | 21.92 | 25.43 |
| Group 3 | 4.68 | 20.98 | 24.25 |
| Group 4 | 4.90 | 21.14 | 24.58 |

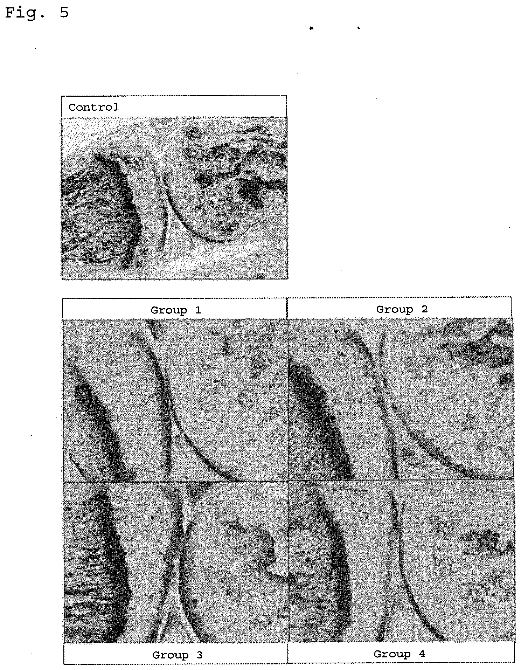

Fig. 5 shows images (representative examples) of each group after Safranin O staining.

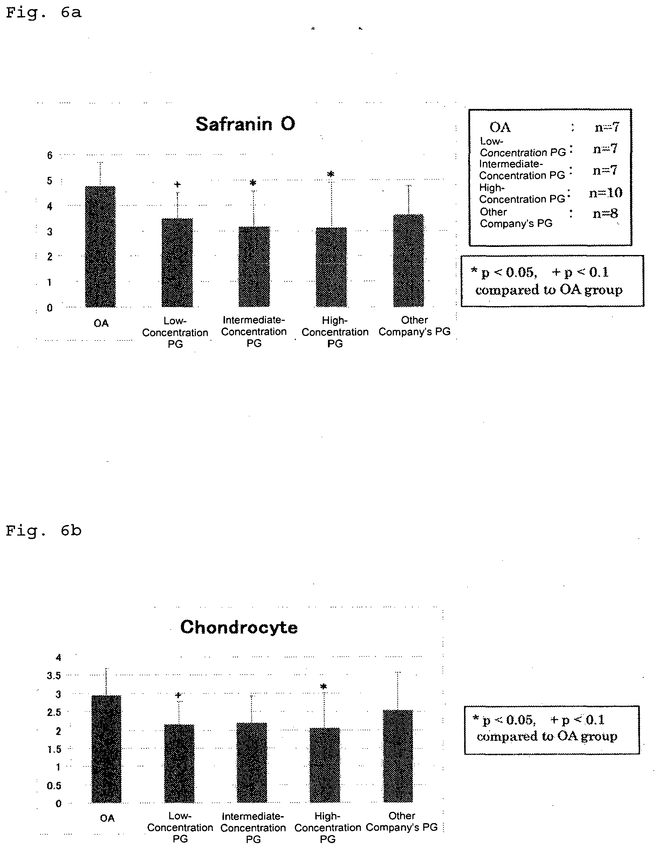

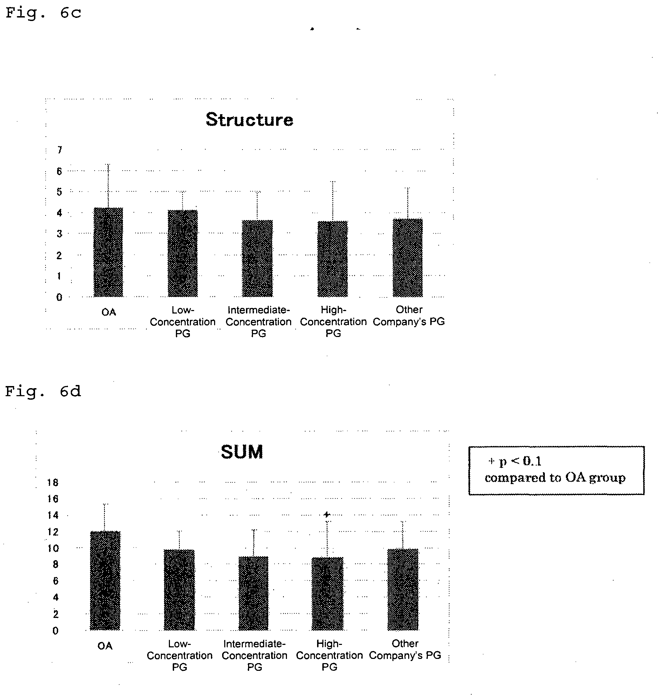

Figs. 6a to 6d show analysis results of scoring each group in terms of the above three items (by three assessors) using the Mankin score, based on the histological images. In Figs. 6a to 6d, OA shows the results of the control, Low-Concentration PG shows the results of group 1, Intermediate-Concentration PG shows the results of group 2, High-Concentration PG shows the results of group 3, and Other Company's PG shows the results of group 4. Fig. 6a shows the analysis results of "Safranin O." Fig. 6b shows analysis results of "Chondrocyte" (the number of chondrocytes). Fig. 6c shows analysis results of "Structure" (cartilage surface structure). Fig. 6d shows the results of analyzing the total score of these three items. The description about the number n of the mice in Fig. 6a also applies to Figs. 6b to 6d.

These results confirmed that Sample No. 1 significantly suppressed cartilage lesion development of osteoarthritis model mice, whereas Sample No. 2 did not exhibit cartilage lesion inhibitory effects.

These results show that proteoglycans having low molecular weights are ineffective for osteoarthritis through oral intake, whereas proteoglycans having molecular weights of not less than 1,800,000 (particularly preferably not less than 5,000,000) can prevent or treat osteoarthritis through oral intake.

## Claims

1. A composition for use in preventing or treating osteoarthritis, comprising a fish cartilage water extract containing proteoglycans having molecular weights of not less than 5,000,000,
wherein the amount of uronic acids derived from the proteoglycans having molecular weights of not less than 5,000,000 accounts for at least 30 mass% of the total uronic acid content of the fish cartilage water extract, and the amount of uronic acids derived from the proteoglycans having molecular weights of not less than 1,800,000 accounts for at least 50 mass% of the total uronic acid content of the fish cartilage water extract,
wherein the composition is for oral administration.

2. The composition for use according to claim 1, wherein the fish cartilage water extract is a hot-water extract of fish cartilage.

3. The composition for use according to claim 1 or claim 2, wherein the fish cartilage is salmon cartilage or trout cartilage.

## Patentansprüche

1. Zusammensetzung zur Verwendung in der Vorbeugung oder Behandlung von Osteoarthritis, umfassend einen Fischknorpelwasserextrakt, der Proteoglykane mit Molekulargewichten von nicht weniger als 5.000.000 enthält,
wobei die Menge von Uronsäuren, die von den Proteoglykanen mit Molekulargewichten von nicht weniger als 5.000.000 abgeleitet sind, mindestens 30 Masse-% des gesamten Uronsäuregehalts des Fischknorpelwasserextrakts ausmacht, und die Menge von Uronsäuren, die von Proteoglykanen mit Molekulargewichten von nicht weniger als 1.800.000 abgeleitet sind, mindestens 50 Masse-% des gesamten Uronsäuregehalts des Fischknorpelwasserextrakts ausmacht,
wobei die Zusammensetzung zur oralen Verabreichung bestimmt ist.

2. Zusammensetzung zur Verwendung nach Anspruch 1, wobei der Fischknorpelwasserextrakt ein Heißwasserextrakt von Fischknorpel ist.

3. Zusammensetzung zur Verwendung nach Anspruch 1 oder Anspruch 2, wobei der Fischknorpel Lachsknorpel oder Forellenknorpel ist.

## Revendications

1. Composition à utiliser dans la prévention ou le traitement de l'ostéoarthrite, comprenant un extrait aqueux de cartilage de poisson contenant des protéoglycanes ayant des masses moléculaires non inférieures à 5 000 000,
dans laquelle la quantité d'acides uroniques tirés des protéoglycanes ayant des masses moléculaires non inférieures à 5 000 000 compte pour au moins 30 % en masse de la teneur totale en acide uronique de l'extrait aqueux de cartilage de poisson, et la quantité d'acides uroniques tirés des protéoglycanes ayant des masses moléculaires non inférieures à 1 800 000 compte pour au moins 50 % en masse de la teneur totale en acide uronique de l'extrait aqueux de cartilage de poisson,
dans laquelle la composition est pour une administration orale.

2. Composition à utiliser selon la revendication 1, dans laquelle l'extrait aqueux de cartilage de poisson est un extrait aqueux chaud de cartilage de poisson.

3. Composition pour l'utilisation selon la revendication 1 ou la revendication 2, dans laquelle le cartilage de poisson est du cartilage de saumon ou du cartilage de truite.
